(19) Europäisches Patentamt
European Patent Office
Office européen des brevets

(11) EP 2 397 473 A1

(12) EUROPEAN PATENT APPLICATION

(43) Date of publication:
**21.12.2011 Bulletin 2011/51**

(21) Application number: **10165878.9**

(22) Date of filing: **14.06.2010**

(51) Int Cl.:
***C07D 263/20*** *(2006.01)* ***A61K 31/421*** *(2006.01)*
***A61P 9/10*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **LEK Pharmaceuticals d.d.**
**1526 Ljubljana (SI)**

(72) Inventor: **The designation of the inventor has not yet been filed**

(74) Representative: **Kunic Tesovic, Barbara**
**Lek Pharmaceuticals d.d.**
**Sandoz Development Center Slovenia - Patents**
**Verovskova 57**
**1526 Ljubljana (SI)**

(54) **A stable highly crystalline anacetrapib**

(57) The present invention relates to stable highly crystalline forms of anacetrapib, to processes for their preparation and to pharmaceutical compositions containing them.

EP 2 397 473 A1

## Description

### Field of the invention

The present invention relates to a highly crystalline stable forms of anacetrapib, to processes for the preparation and to pharmaceutical compositions containing such forms.

### Background of the invention

The compound (4S,5R)-5-[3,5-bis(trifluoromethyl)phenyl]-3-[[2-(4-fluoro-2-methoxy-5-propan-2-ylphenyl)-5-(trifluoromethyl)phenyl]methyl]-4-methyl-1,3-oxazolidin-2-one, also named 5(R)-[3,5-bis(trifluoromethyl)phenyl]-3-[4'-fluoro-5'-isopropyl-2'-methoxy-4-(trifluoromethyl)biphenyl-2-ylmethyl]-4(S)-methyloxazolidin-2-one, and briefly named anacetrapib (Formula I) has been shown to act as an inhibitor of cholesterylester transfer protein (CETP).

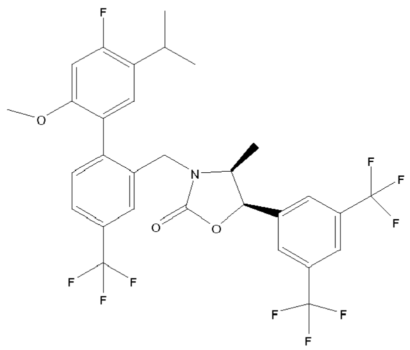

Formula I

WO06/014413 generally relates to CETP inhibitors used for the prevention and treatment of atherosclerosis, comprising anacetrapib and pharmaceutically acceptable salts thereof and discloses the process for its preparation. In WO06/014413 anacetrapib is obtained as a clear glass. WO06/014413 is silent as to the nature of specific crystalline forms of anacetrapib.

WO07/005572 describes a process for synthesizing CETP inhibitors, comprising anacetrapib. A heptane solvate and a non-solvate form of anacetrapib which are said to be crystalline are mentioned. Initially obtained crystalline heptane solvate de-solvates at room temperature under a flow of nitrogen or air, or under vacuum, while the crystalline non-solvate form of anacetrapib is said to slowly convert to the amorphous form on standing at room temperature. Furthermore, the use of a pharmaceutical composition comprising crystalline anhydrous form in the manufacture of a medicament for the treatment or prevention of atherosclerosis is described.

The fact that in WO06/014413 a glassy amorphous solid was obtained, and that only later in WO07/005572 crystalline forms are vaguely mentioned suggests that anacetrapib is a compound that is difficult to crystallize, and if initially obtained at all is readily transformed into amorphous form, hence is unstable. The results of our own research confirmed this hypothesis.

Crystallization is the most important purification process for pharmaceutical compounds with molecular weights from about 200 to 1200 g/mol. However, crystallization is a critical process. This is particularly the case when a given substance is difficult to crystallize, as this is the case for anacetrapib. The amorphous form can be present as a phase impurity in a crystalline sample, and for compounds that are difficult to crystallize, it is possible that the content of amorphous form may be from about 10% to 20%, or more. If a given crystallization process leads to a product with varying amounts of amorphous form, even if present in a predominantly crystalline material, this may lead to a nonuniform pharmaceutical drug product due to the large solubility difference between the crystalline and the amorphous state (the amorphous form of a given small molecule substance typically shows a much greater aqueous solubility than all existing crystalline forms). Therefore, it is important to provide stable crystalline forms that can be produced with low levels of amorphous form.

Polymorphism is defined as the ability of a substance to crystallize in more than one crystal lattice arrangement. Polymorphism can influence different aspects of solid state properties of a drug. Different crystal forms of a substance may differ considerably from one another in many respects such as their crystallinity, stability and/or dissolution rate, solubility, hygroscopicity, crystal habits, bioavailability and formulation handling characteristics. The discovery of novel polymorphic forms of a pharmaceutically useful compound provides a new opportunity to improve the performance

characteristics of a pharmaceutical product.

In general, when a compound is used as a pharmaceutical product, chemical and physical stability of the compound is required so as to maintain quality and/or facilitate preservation. Not only the final pharmaceutical composition but also a compound as a synthetic starting material is desirably chemically and physically stable for the same reasons. As becomes apparent, this is a challenging task particularly in the case of anacetrapib.

There is an unmet need in the art and therefore it was an object to provide a highly crystalline form of anacetrapib, in particular crystalline polymorphic forms, with better thermal stability that offer advantages for anacetrapib purification processes and for preparing reproducible pharmaceutical formulations with longer shelf-lives. Furthermore, there is a need to provide a stable highly crystalline form, in particular crystalline polymorphic forms, in an aimed and reproducible manner, thereby allowing to obtain a desired specific, predetermined form in a good purity.

## Summary of the invention

The present invention provides the following items including main aspects and preferred embodiments, which alone and in combination particularly contribute to solving the above object and eventually provide additional advantages, respectively:

1. Anacetrapib which is defined by a stable highly crystalline form.

2. The anacetrapib according to item 1, characterized by at least one, preferably at least three, more preferably all of the characteristic peaks in the X-ray powder diffractogram at 2θ values of 5.1, 7.7, 19.4, 20.7 and 21.2, respectively exactly or ± 0.2 degrees 2θ at the indicated 2θ values.

3. The anacetrapib according to item 1, characterized by at least one, preferably at least three, more preferably all of the characteristic peaks in the X-ray powder diffractogram at 2θ values of 5.1, 7.7, 14.3, 17.0, 17.9, 19.4, 20.7, 21.2 and 22.1, respectively exactly or ± 0.2 degrees 2θ at the indicated 2θ values.

4. The anacetrapib according to item 1, characterized by the X-ray powder diffractogram as shown in Figure 1.

5. The anacetrapib according to any one of items 1 to 4, with at least three powder X-ray diffraction peaks with a signal to noise ratio greater than 50 : 1.

6. The anacetrapib according to any one of items 1 to 5, further characterized by differential scanning calorimetry thermogram having endotherm peak in the range of 75°C to 95 °C.

7. The anacetrapib according to any one of items 1 to 6 with an enthalpy of fusion greater than 22 J/g.

8. A process for the preparation of the anacetrapib according to any one of preceding items, comprising the steps of:

   a) dissolving of amorphous form of anacetrapib in a solvent;
   b) adding an anti-solvent to the mixture obtained in step a);
   c) optionally stirring the mixture obtained in step b);
   d) allowing the mixture obtained in step b) to crystallize.

9. The process according to item 8, wherein step a) is carried out in a solvent, selected from

   - organic solvent, preferably alcohol, more preferably ethanol and
   - a mixture of organic solvent and water, preferably a mixture of alcohol and water, most preferably a mixture of ethanol and water.

10. The process according to item 8 or 9, wherein the solvent is a mixture of organic solvent and water at a ratio of organic solvent, preferably alcohol and more preferably ethanol, to water is about 4:1.

11. The process according to any one of items 8 to 10, wherein an anti-solvent in step b) is water.

12. The process according to any one of items 8 to 11, wherein the mixture obtained in step b) is optionally cooled to a temperature lower than room temperature.

13. The process according to any one of items 8 to 12, wherein seed crystals of the anacetrapib according to any one of items 1 to 7 are added to the mixture obtained in step b).

14. A pharmaceutical composition comprising crystalline anacetrapib according to any one of items 1 to 7.

15. The pharmaceutical composition according to item 14, for the use in prophylaxis or therapeutic treatment of atherosclerosis in a patient, wherein the anacetrapib is in a crystalline non-solvate form.

16. The anacetrapib according to item 1, optionally containing 0 to 30% (w/w) of solvent included in the crystal lattice.

17. The anacetrapib according to item 16, wherein the included solvent is a linear alkane, preferably n-pentane.

18. The anacetrapib according to item 16 or 17, characterized by at least one, preferably at least three, more preferably all of the characteristic peaks in the X-ray powder diffractogram at 2θ values of 17.5, 20.2, 20.6, and 20.9, respectively exactly or ± 0.2 degrees 2θ at the indicated 2θ values.

19. The anacetrapib according to item 16 or 17, characterized by at least one, preferably at least three, more preferably all significant peaks in the X-ray powder diffractogram at 2θ values of 7.4, 14.2, 17.5, 20.2, 20.6, 20.9, 22.3 and 22.6, respectively exactly or ± 0.2 degrees 2θ at the indicated 2θ values.

20. The anacetrapib according item 16 or 17, characterized by the X-ray powder diffractogram as shown in Figure 4.

21. The anacetrapib according to item 16, wherein the included solvent is nonafluoro-methoxy-butane.

22. The anacetrapib according to item 16 or 21, characterized by at least one, preferably at least three, more preferably all of the characteristic peaks in the X-ray powder diffractogram at 2θ values of 6.0, 14.0, 16.6, 18.1 and 19.5, respectively exactly or ± 0.2 degrees 2θ at the indicated 2θ values.

23. The anacetrapib according to item 16 or 21, characterized by at least one, preferably at least three, more preferably all of the characteristic peaks in the X-ray powder diffractogram at 2θ values of 6.0, 13.7, 14.0, 16.6, 18.1, 19.5, 20.6 and 27.5, respectively exactly or ± 0.2 degrees 2θ at the indicated 2θ values.

24. The anacetrapib according to item 16 or 21, characterized by the X-ray powder diffractogram as shown in Figure 8.

25. Use of the anacetrapib according to any one of items 16 to 24 for producing a pharmaceutically active anacetrapib to be used in a pharmaceutically composition.

26. Crystalline anacetrapib characterized by at least one, preferably at least three, more preferably all of the characteristic peaks in the X-ray powder diffractogram at 2θ values of 5.1, 7.7, 19.4, 20.7 and 21.2, respectively exactly or ± 0.2 degrees 2θ at the indicated 2θ values.

27. Crystalline anacetrapib characterized by at least one, preferably at least three, more preferably all of the characteristic peaks in the X-ray powder diffractogram at 2θ values of 5.1, 7.7, 14.3, 17.0, 17.9, 19.4, 20.7, 21.2 and 22.1, respectively exactly or ± 0.2 degrees 2θ at the indicated 2θ values.

28. Crystalline anacetrapib characterized by the X-ray powder diffractogram as shown in Figure 1.

29. The anacetrapib according to any one of items 26 to 28 with at least three powder X-ray diffraction peaks with a signal to noise ratio greater than 50 : 1.

30. The anacetrapib according to any one of items 26 to 29, further characterized by differential scanning calorimetry thermogram having endotherm peak in the range of 75 °C to 95 °C.

31. The anacetrapib according to any one of items 26 to 30 with an enthalpy of fusion greater than 22 J/g.

32. A process for the preparation of the anacetrapib according to any one of items 25 to 30, comprising the steps of:

a) dissolving of amorphous form of anacetrapib in a solvent;

b) adding an anti-solvent to the mixture obtained in step a);
c) optionally stirring the mixture obtained in step b);
d) allowing the mixture obtained in step b) to crystallize.

33. The process according to item 32, wherein step a) is carried out in a solvent, selected from

- organic solvent, preferably alcohol, more preferably ethanol and
- a mixture of organic solvent and water, preferably a mixture of alcohol and water, most preferably a mixture of ethanol and water.

34. The process according to items 32 or 33, wherein the solvent is a mixture of organic solvent and water at a ratio of organic solvent, preferably alcohol and more preferably ethanol, to water is about 4 : 1.

35. The process according to any one of items 32 to 34, wherein an anti-solvent in step b) is water.

36. The process according to any one of items 32 to 35, wherein the mixture obtained in step b) is optionally cooled to a temperature lower than room temperature.

37. The process according to any one of items 32 to 36, wherein seed crystals of the crystalline anacetrapib according to any one of items 25 to 30 are added to the mixture obtained in step b).

38. A pharmaceutical composition comprising crystalline anacetrapib according to any one of items 26 to 31.

39. The pharmaceutical composition according to item 38, for the use in prophylaxis or therapeutic treatment of atherosclerosis, wherein the anacetrapib is a crystalline non-solvate.

40. Crystalline anacetrapib optionally containing 0 to 30% (w/w) of solvent included in the crystal lattice.

41. The anacetrapib according to item 40, wherein the included solvent is n-pentane.

42. The anacetrapib according to item 40 or 41, characterized by at least one, preferably at least three, more preferably all of the characteristic peaks in the X-ray powder diffractogram at 2θ values of 17.5, 20.2, 20.6, and 20.9, respectively exactly or ± 0.2 degrees 2θ at the indicated 2θ values.

43. The anacetrapib according to item 40 or 41, characterized by at least one, preferably at least three, more preferably all significant peaks in the X-ray powder diffractogram at 2θ values of 7.4, 14.2, 17.5, 20.2, 20.6, 20.9, 22.3 and 22.6, respectively exactly or ± 0.2 degrees 2θ at the indicated 2θ values.

44. The anacetrapib according item 40 or 41, characterized by the X-ray powder diffractogram as shown in Figure 4.

45. The anacetrapib according to item 40, wherein the included solvent is nonafluoro-methoxy-butane.

46. The anacetrapib according to item 40 or 45, characterized by at least one, preferably at least three, more preferably all of the characteristic peaks in the X-ray powder diffractogram at 2θ values of 6.0, 14.0, 16.6, 18.1 and 19.5, respectively exactly or ± 0.2 degrees 2θ at the indicated 2θ values.

47. The anacetrapib according to item 40 or 45, characterized by at least one, preferably at least three, more preferably all of the characteristic peaks in the X-ray powder diffractogram at 2θ values of 6.0, 13.7, 14.0, 16.6, 18.1,19.5, 20.6 and 27.5, respectively exactly or ± 0.2 degrees 2θ at the indicated 2θ values.

48. The anacetrapib according to item 40 or 45, characterized by the X-ray powder diffractogram as shown in Figure 8.

49. The anacetrapib according to item 40, wherein the included solvent is methyl-cyclohexane solvate.

50. The crystalline anacetrapib according item 49, characterized by the X-ray powder diffractogram as shown in Figure 9.

51. Use of the anacetrapib according to any one of items 40 to 50 for producing a pharmaceutically active anacetrapib

to be used in a pharmaceutically composition.

The present invention provides a new crystalline anacetrapib with good thermal stability that is easy to handle and has a high degree of purity. Moreover, the present invention provides a process for repeatedly obtaining the said crystalline anacetrapib. In addition, the present invention relates to a new highly crystalline forms of anacetrapib stable at room temperature and thus easy to handle. The present invention further provides novel crystalline polymorphic forms of anacetrapib. The present invention provides advantages for preparing reproducible pharmaceutical formulations compared to amorphous substances or compared to conventional materials which are unstable and readily and substantially transformed into amorphous form.

## Detailed description of the invention

In the following, the present invention will be described in more details by preferred embodiments and examples while referring to the attached drawings, noting however, that these embodiments, examples and drawings are presented for illustrative purposes only and shall not limit the invention in anyway.

Figure 1 is an X-Ray powder diffractogram of Form B according to a preferred embodiment of the present invention;
Figure 2 is a differential scanning calorimetry thermogram of Form B according to a preferred embodiment of the present invention;
Figure 3 is an X-Ray powder diffractogram of Form B after preparation on day 1 (lower line) and on day 32 (upper line) according to Example 5 of the present invention;
Figure 4 is an powder X-ray diffraction pattern of Form C according to Example 7 of the present invention;
Figure 5 is an powder X-ray diffraction pattern of Form C according to Example 8 of the present invention;
Figure 6 is a differential scanning calorimetry thermogram of Form C according to a preferred embodiment of the present invention;
Figure 7 is an powder X-ray diffraction pattern of Form C after preparation (bottom line) and after storage for 20 days at room temperature (upper line) according to Example 10 of the present invention;
Figure 8 is an X-Ray powder diffractogram of Form D according to Example 11 of the present invention;
Figure 9 is an X-Ray powder diffractogram of Form D according to Example 12 of the present invention;
Figure 10 is an X-ray powder diffractogram of Form D after preparation on day 1 (lower line) and after storage for two weeks at room temperature (upper line) according to Example 13 of the present invention.

According to one aspect, the present invention discloses novel stable, highly crystalline forms of anacetrapib, designated herein as Form B, Form C and Form D. Crystalline forms of anacetrapib according to the present invention have a high h degree of crystallinity, great thermal stability which is indicative for improved chemical and/or physical stability, and show no changes in polymorphic forms. The present invention also provides a simple, effective and economically advantageous process for preparation of crystalline forms of anacetrapib. Furthermore, crystalline forms of anacetrapib due to their excellent stability are easy to handle.

The term "highly crystalline" used herein generally means having a signal-to-noise ratio of the most intense peak at least 100 : 1 of and having a signal-to-noise ratio of the second most intensive peak of at least 50 : 1.

A highly crystalline sample of anacetrapib is characterized by a low level of amorphous content (typically up to only 20 % at maximum), several intense powder X-ray diffraction peaks and a reasonably high enthalpy of fusion determined by DSC. Amorphous anacetrapib has a glass transition temperature near 40 °C and no melting peak is observed (*i.e.* the enthalpy of fusion equals zero). Amorphous and poorly crystalline anacetrapib exhibits an enthalpy of fusion from 0 to 20 J/g, whereas highly crystalline anacetrapib shows an enthalpy of fusion greater than 22 J/g, preferably about 25 to 30 J/g. In addition, highly crystalline anacetrapib shows at least three powder X-ray diffraction peaks with a signal-to-noise ratio that is greater than 50 : 1.

The term "stable" used herein generally means having about the same characteristics determined by X-ray powder diffraction (XPD) after standing in a closed vial at room temperature at about 19 °C to 25 °C for at least one week, preferably at least two weeks, more preferably more than three weeks compared to day 1.

The term "same characteristics" used herein generally means having XRD peak locations within the given range of ± 0.2° in 2 theta angle, having XRD peak intensities within 50%, suitably within 30% and particularly within 20% of the original value before storage, and having at least three peaks with a signal-to-noise ratio greater than 50 in XRD pattern.

The term "about" used herein generally means within 10%, suitably within 5% and particularly within 1 % of a given value or range, or within 10% of a given intensity value or ratio. Alternatively, the term "about" means within an acceptable standard error of the mean, when considered by one of the ordinary skill in the art.

Anacetrapib is a compound that is extremely difficult to crystallize. Surprisingly, the crystalline forms of anacetrapib according to the present invention, compared to previously described non-solvate or heptane solvate forms, show a good tendency towards crystallization. Therefore the forms described herein are very useful for the purification of the compound, even if the compound as such is optionally formulated in the amorphous form. Furthermore the new crystalline forms of anacetrapib are thermodynamically substantially more stable thus are likely to display higher chemical and/or physical stability. The crystalline forms described herein may be useful of its own as final pharmaceutically acceptable forms, or may be used as intermediates for being transformed onto other forms, including but not limited to amorphous form, to mixtures of different forms (amorphous and/or different crystalline forms) or to individual forms. Yet, the present invention can significantly contribute reproducibility and product uniformity.

In certain embodiments, the present invention relates to Form B, characterized by one of the following particular XRD patterns:

in a sufficiently pure and unique form, Form B is characterized by at least significant peaks at 2θ values of 5.1, 7.7, 19.4, 20.7 and 21.2 in an XRD, respectively exactly or ± 0.2 degrees 2θ at the indicated 2θ values;
in a more specific form, Form B is characterized by peaks at 2θ values of 5.1, 7.7, 14.3, 17.0, 17.9, 19.4, 20.7, 21.2 and 22.1 in an XRD, respectively exactly or ± 0.2 degrees 2θ at the indicated 2θ values;
in a certain exemplified form, the crystalline Form B is characterized by an X-ray powder diffractog ram shown in Figure 1.

Unexpectedly, the said polymorphic Form B is further characterized as being highly crystalline (*i.e.* having a signal-to-noise ratio of at least about 100 : 1 of the most intense peak and having a signal-to-noise ratio of at least about 50 : 1 of the second and third most intensive peaks if measured as indicated in the experimental part). Surprisingly, the said polymorphic Form B is further characterized as being stable (*i.e.* having about the same characteristics after standing at room temperature at about 19 °C to 25 °C for at least one week, preferably at least two weeks, more preferably more than three weeks compared to day 1). In particular, the characteristics of Form B are characterized by XRD having at least significant peaks at 2θ values of 5.1, 7.7, 19.4, 20.7 and 21.2, respectively exactly or ± 0.2 degrees 2θ at the indicated 2θ values, preferably peaks in the XRD at 2θ values of 5.1, 7.7, 14.3, 17.0, 17.9, 19.4, 20.7, 21.2 and 22.1, respectively exactly or ± 0.2 degrees 2θ at the indicated 2θ values, more preferably the XRD as shown in Figure 1 after standing at room temperature at about 19 °C to 25 °C for at least one week, preferably at least two weeks, more preferably more than three weeks.

In another aspect the invention relates to a polymorphic Form B further characterized by a differential scanning calorimetry thermogram having an endothermic peak in the range of 75 °C to 95 °C, preferably in the range of 85 °C to 90 °C.

Another aspect of the present invention relates to a process for the preparation of polymorphic Form B, the process comprising the steps of:

a) dissolving of amorphous form of anacetrapib in a solvent;
b) adding an anti-solvent to the mixture obtained in step a);
c) optionally stirring the mixture obtained in step b);
d) allowing the mixture obtained in step b) to crystallize.

This aspect of the present invention provides an efficient combination of process steps to repeatedly arrive at pure and physically stable Form B of anacetrapib. Formation of Form B according to this aspect of the present invention may constitute parts of a complete process, *i.e.* involving consecutive steps which start from relevant educts and finally yield the desired Form B of anacetrapib.

The process includes dissolving of amorphous anacetrapib in a solvent and allowing the mixture obtained to crystallize. A solvent is selected from an organic solvent, preferably alcohol, more preferably ethanol or a mixture of organic solvent and water, preferably a mixture of alcohol and water, most preferably a mixture of ethanol and water is used for dissolving the amorphous form of anacetrapib.

Form B of anacetrapib can be isolated or recovered from the reaction solution by precipitation, while allowing the desired crystal form to crystallize. The precipitation can be spontaneous depending on solvent system. The precipitation is caused by adding an anti-solvent, for example water, ethers and hydrocarbons.

In one process embodiment of the invention the precipitation of Form B occurs after adding an anti-solvent, preferably water to the reaction mixture and after long standing of the suspension at temperature lower than room temperature, preferably at about 5 °C, while optionally stirring.

In a preferred process embodiment of the invention the precipitation of Form B occurs after adding water to a solution of the compound in an alcohol, preferably ethanol to a solvent to water ratio of about 4 : 1, while optionally stirring and adding seed crystals of Form B.

In another process embodiment of the invention the precipitation of Form B occurs after adding an anti-solvent,

preferably water to the reaction mixture, while optionally stirring and adding seed crystals of Form B (obtained according to aforedefined aspect of the invention) to the reaction mixture and after long standing of the suspension at about room temperature at about 19 °C to 25 °C.

Induction of the precipitation can be further enhanced by reducing the temperature of reaction mixture, especially if initial temperature of reaction mixture is elevated. Induction of the precipitation can also be enhance by reduction of solution volume, preferably under diminished pressure, or by complete evaporation of solvent.

Obtained Form B of anacetrapib may be separated by techniques well known in the art, *e.g.* filtration, centrifugation, decanting, preferably by filtration under vacuum at room temperature at about 19 °C to 25 °C.

This aspect of the present invention provides particularly efficient combination of process steps to repeatedly arrive at pure and physically stable Form B of anacetrapib. The process allows the desired crystalline anacetrapib to be easily and repeatedly purified. In addition, the Form B of the present invention may be obtained substantially free of any residual organic solvents, that is preferably less than 500 parts of organic solvent per million (ppm), more preferably less than 100 parts of organic solvent per million (ppm).

Another aspect of the present invention is a pharmaceutical composition for administering a therapeutically effective amount of crystal Form B of anacetrapib of present invention in unit dosage form.

Form B of anacetrapib in accordance with present invention can be embodied for example in form of tablet, capsules, pellets, granules and suppositories or their combined forms. Pharmaceutical composition in accordance with present invention can be suitable for immediate release or modified release of crystal Form B of anacetrapib of the present invention. Solid pharmaceutical compositions can be for example coated with aim of increasing peletibility or regulating the disintegration or absorption.

Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of lactose, microcrystalline cellulose, cellulose derivatives, (*e.g.* hydroxypropylcellulose, croscarmellose sodium), polyacrylates, calcium carbonate, starch, colloidal silicone dioxide, sodium starch glycolate, talc, magnesium stearate, mannitol, polyvinylpyrrolidone, polyethylene glycol and other excipients known in the field of the pharmaceutical technology.

Optionally, the pharmaceutical compositions of the invention may be combination product comprising one or more additional pharmaceutically active components in addition to Form B of anacetrapib.

In another aspect the present invention is related to use of crystal Form B of anacetrapib, for the manufacture of medicament for inhibiting of cholesterylester transfer protein in an organism. Said inhibitor is preferably used in prophylaxis or therapeutic treatment of atherosclerosis in a patient in need of treatment comprising the administration of a therapeutically effective amount of anacetrapib, characterized as being a crystalline non-solvate.

In another aspect, the present invention relates to new cristalline forms of anacetrapib (Form C and Form D) that may exist in a solvent-free state, but can optionally contain up to about 30% of the respective solvent in the crystal lattice.

In a particular aspect the present invention relates to a crystalline anacetrapib (Form C), which can preferably contain n-pentane or other linear alkanes. Compared to previously described non-solvate or heptane solvate forms, the crystalline Form C according to present invention is thermodynamically more stable and thus is likely to display higher chemical and/or physical stability. Whereas isolation of these crystalline solvates is difficult, and especially difficult when small quantities are used in a crystallization experiment, it is conceivable that some of the suspected solvated forms could be helpful to drug substance purification. Form C is particularly useful for the purification process because pentane can be easily removed.

In particular embodiment, the present invention relates to Form C, characterized by XRD. In a sufficiently pure and unique form, Form C is characterized by at least XRD significant peaks at 2θ values of 17.5, 20.2, 20.6, and 20.9, respectively exactly or ± 0.2 degrees 2θ at the indicated 2θ values,

in a more pure and specific form, Form C is characterized by peaks at 2θ values of 7.4, 14.2, 17.5, 20.2, 20.6, 20.9, 22.3 and 22.6 in an XRD, respectively exactly or ± 0.2 degrees 2θ at the indicated 2θ values;

in a certain exemplified form, the crystalline Form C is characterized by an X-ray powder diffractogram shown in Figure 4.

Unexpectedly, the said polymorphic Form C is further characterized as being highly crystalline (*i.e.* having a signal-to-noise ratio of at least about 100 : 1 of the most intense peak and having a signal-to-noise ratio of at least about 50 : 1 of the second and third most intensive peaks if measured as indicated in the experimental part). Surprisingly, the said Form C is further characterized as being stable (*i.e.* having about the same characteristics after standing at room temperature at about 19 °C to 25 °C for at least one week, preferably at least two weeks, more preferably more than three weeks compared to day 1). In particular, the characteristics of Form C are characterized by XRD having at least significant peaks at 2θ values of 17.5, 20.2, 20.6, and 20.9, respectively exactly or ± 0.2 degrees 2θ at the indicated 2θ values, preferably peaks in the XRD at 2θ values of 7.4, 14.2, 17.5, 20.2, 20.6, 20.9, 22.3 and 22.6, respectively exactly or ± 0.2 degrees 2θ at the indicated 2θ values, more preferably the XRD as shown in Figure 4 after standing at room

temperature at about 19 °C to 25 °C for at least one week, preferably at least two weeks, more preferably more than three weeks.

Another aspect of the present invention relates to a process for the preparation of crystalline Form C of anacetrapib. The process includes dissolving of amorphous anacetrapib in a solvent and allowing the mixture obtained to crystallize. A solvent is selected from an organic solvent, preferably pentane is used for dissolving the amorphous form of anacetrapib.

This aspect of the present invention provides an efficient combination of process steps to repeatedly arrive at pure and physically stable Form C of anacetrapib. Formation of Form C according to this aspect of the present invention may constitute parts of a complete process, *i.e.* involving consecutive steps which start from relevant educts and finally yield the desired Form C of anacetrapib.

Form C of anacetrapib can be isolated or recovered from the reaction solution by precipitation, while allowing the desired crystal form to crystallize. The precipitation can be spontaneous depending on solvent system. Initiation of the precipitation can be enhanced by reducing the temperature of reaction mixture. Initiation of the precipitation can also be enhanced by reduction of solution volume, preferably under diminished pressure, or by complete evaporation of solvent.

In one aspect of the invention the precipitation of Form C occurs after adding a solvent and after long standing of the suspension at temperature lower than room temperature, preferably lower than 5 °C.

Obtained Form C of anacetrapib may be separated by techniques well known in the art, *e.g.* filtration, centrifugation, decanting, evaporation of the remaining solvent, lyophilization, preferably at temperatures lower than room temperature.

This aspect of the present invention provides particularly efficient combination of process steps to repeatedly arrive at pure and physically stable Form C of anacetrapib. The process allows the desired crystalline anacetrapib to be easily and repeatedly purified.

In another aspect the present invention relates to a crystalline anacetrapib (Form D), which can preferably contain methyl-cyclohexane or nonafluoro-methoxy-butane. Compared to previously described non-solvate or crystalline heptane solvate forms, the crystalline Form D according to present invention is thermodynamically more stable and thus is likely to display higher chemical and/or physical stability.

In particular embodiment, the present invention relates to Form D, characterized by XRD. In a sufficiently pure and unique form, Form D is characterized by at least XRD significant peaks at 2θ values of 6.0, 14.0, 16.6, 18.1 and 19.5, respectively exactly or ± 0.2 degrees 2θ at the indicated 2θ values,

in a more pure and specific form, Form D is characterized by peaks at 2θ values of 6.0, 13.7, 14.0, 16.6, 18.1,19.5, 20.6 and 27.5 in an XRD, respectively exactly or ± 0.2 degrees 2θ at the indicated 2θ values;

in a certain exemplified form, the crystalline Form D is characterized by an X-ray powder diffractogram shown in Figure 8.

Unexpectedly, the said polymorphic Form D is further characterized as being highly crystalline (*i.e.* having a signal-to-noise ratio of at least about 100 : 1 of the most intense peak and having a signal-to-noise ratio of at least about 50 : 1 of the second and third most intensive peaks if measured as indicated in the experimental part). Surprisingly, the said Form D is further characterized as being stable (*i.e.* having about the same characteristics after standing at room temperature at about 19 °C to 25 °C for at least one week, preferably at least two weeks, more preferably more than three weeks compared to day 1). In particular, the characteristics of Form D are characterized by XRD having at least significant peaks at 2θ values of 6.0, 14.0, 16.6, 18.1 and 19.5, respectively exactly or ± 0.2 degrees 2θ at the indicated 2θ values, preferably peaks in the XRD at 2θ values of 6.0, 13.7, 14.0, 16.6, 18.1,19.5, 20.6 and 27.5, respectively exactly or ± 0.2 degrees 2θ at the indicated 2θ values, more preferably the XRD as shown in Figure 8 after standing at room temperature at about 19 °C to 25 °C for at least one week, preferably at least two weeks, more preferably more than three weeks.

Another aspect of the present invention relates to a process for the preparation of crystalline Form D of anacetrapib. The process includes dissolving of amorphous anacetrapib in a solvent and allowing the mixture obtained to crystallize. A solvent is selected from an organic solvent, preferably methyl-cyclohexane, most preferably nonafluoro-methoxy-butane is used for dissolving the amorphous form of anacetrapib.

This aspect of the present invention provides an efficient combination of process steps to repeatedly arrive at pure and physically stable Form D of anacetrapib. Formation of Form D according to this aspect of the present invention may constitute parts of a complete process, *i.e.* involving consecutive steps which start from relevant educts and finally yield the desired Form D of anacetrapib.

Form D of anacetrapib can be isolated or recovered from the reaction solution by precipitation, while allowing the desired crystal form to crystallize. The precipitation can be spontaneous depending on solvent system. Initiation of the precipitation can be enhanced by reducing the temperature of reaction mixture. Initiation of the precipitation can also be enhanced by reduction of solution volume, preferably under diminished pressure, or by complete evaporation of solvent.

In one particular process embodiment of the invention the precipitation of Form D occurs after adding a solvent

and after long standing of the suspension at temperature lower than room temperature, preferably at about 5 °C.

Obtained Form D of anacetrapib may be separated by techniques well known in the art, *e.g.* filtration, centrifugation, decanting, evaporation of the remaining solvent, lyophilization, preferably at temperatures lower than room temperature, preferably at about -80 °C.

This aspect of the present invention provides particularly efficient combination of process steps to repeatedly arrive at pure and physically stable Form D of anacetrapib. The process allows the desired crystalline anacetrapib to be easily and repeatedly purified.

**Experimental procedures**

Anacetrapib may be prepared according to procedures in PCT application WO2007/005572.

High-throughput polymorph screening investigations using a set of 96 different solvents and solvent mixtures under 256 different experimental conditions were performed and revealed no new form of crystalline anacetrapib.

Example 1: Preparation of seed crystals for Form B.

12.9 mg of amorphous anacetrapib was weighed into a small 4 ml glass vial and dissolved in 50 microliters (μl) of ethanol : water 95 : 5 (v/v) and 200 μl of ethanol were added. To this solution 100 μl of water was added and a slightly turbid suspension was obtained. This suspension was stirred at room temperature at about 19 °C to 25 °C for about one day. Thereafter, the vial was stored in a refrigerator at 5 ± 2 °C for about five weeks. Light microscopic inspection of the solid formed in the stored vial showed that crystalline material was obtained. Yield is about 10 mg.

Example 2: Preparation of seed crystals for Form B.

154 mg of amorphous anacetrapib was weighed into a small 7 ml glass vial and dissolved in 1.0 ml of ethanol. To this solution 200 μl of water was added and a turbid suspension was obtained. This suspension was placed on a laboratory shaker and shaken at 500 rpm for about one day. As no crystallization was observed, the vial was stored in a refrigerator at 5 ± 2 °C. Visual inspection of the glass vial after eight days did not reveal any sign of crystalline material. However, after five additional weeks of storage of the sample in the refrigerator at 5 ± 2 °C the visual appearance of the sample suggested a crystalline material. Light microscopic inspection of the solid formed in the stored vial confirmed that crystalline material was obtained. Investigation of the obtained product by powder X-ray diffraction showed essentially the same XRD pattern as shown in Figure 1. The yield was about 100 mg.

Example 3: Preparation of Form B using seed crystals.

282 mg of amorphous anacetrapib was weighed into a 15 ml glass vial and dissolved in 4.0 ml of ethanol. To this solution 1.0 ml water was added and upon shaking of the vial a slightly turbid emulsion (or suspension) was obtained. To this emulsion 4 to 5 mg of seed crystals of Form B obtained by a process according to Example 1 or Example 2, were added and the mixture was stirred at room temperature at about 19 °C to 25 °C for two days. Thereafter, a suspension was obtained which was filtered and the obtained solid material was dried under vacuum at room temperature at about 19 °C to 25 °C for about twenty hours. Investigation of the product by powder X-ray diffraction showed that a crystalline form was obtained as shown in Figure 1. DSC of this sample showed an endothermic signal attributable to melting of the sample near 87 °C with an enthalpy of fusion of about 26 J/g as shown in Figure 2. Thermogravimetric analysis of the obtained sample showed a weight loss of less than 0.1% upon heating to 150 °C at a heating rate of 10 K/min. The residual content of ethanol was measured by gas chromatography coupled with mass spectrometry. The amount of residual ethanol was found to be less than 100 ppm. The yield was about 160 mg.

The signal to noise ratios of the strongest peaks of the powder X-ray diffraction pattern were evaluated and the following results were found:

| Peak location (2θ) | 5.1° | 7.7° | 19.4° | 21.2° |
|---|---|---|---|---|
| Peak counts | 3607 | 15017 | 6397 | 9448 |
| Baseline counts | 521 | 772 | 2340 | 2309 |
| Noise level counts | 80 | 80 | 80 | 80 |
| Signal to noise | 77 | 356 | 101 | 178 |

Example 4: Preparation of Form B.

About 1.1 g of amorphous anacetrapib was dissolved in 20 ml of ethanol and 5 ml water was added as anti-solvent. The solution was stirred for about 30 minutes before about 5 mg of seed crystals of anacetrapib Form B were added. Stirring was continued for six days at room temperature until crystallization was observed by light microscopy. Stirring was continued and nitrogen was purged through the vial at a flow rate of 50 ml/min in order to remove solvent to a volume of about 15 ml. After two days of further stirring under nitrogen the suspension was filtered and the obtained solid dried under vacuum at room temperature at about 19 °C to 25 °C for about 4 hours. Powder X-ray diffraction of the obtained sample shows that Form B was obtained with yield of 831 mg.

Example 5: Solid-state stability of Form B at room temperature.

42 mg of crystalline Form B were placed into a 4 ml glass vial with its screw cap closed and stored for 32 days at room temperature. Thereafter, powder X-ray diffraction of the stored sample was performed and essentially the same XRD pattern as shown in Figure 3 (upper and lower line) was obtained. Five months later the sample recovered from the X-ray sample holder was investigated by light microscopy and it was still found to be crystalline.

Example 6: Solid-state stability of Form B at about 40 °C.

43 mg of crystalline Form B were placed into a 4 ml glass vial with its screw cap closed and stored for 32 days at 40 °C. Thereafter, powder X-ray diffraction of the stored sample was performed and essentially the same XRD pattern as shown in Figure 3 (upper and lower line) was obtained.

Example 7: Preparation of Form C (with n-pentane).

687 mg amorphous anacetrapib was weighed into a 15 ml glass vial and dissolved in 3.0 ml pentane at room temperature. This solution was slowly cooled to 4 °C and stirred at this temperature. After about three hours a suspension was obtained and stirring was continued overnight. On the next day the temperature was reduced to -5 °C and stirring was continued for two hours before the solid product was separated by filtration with a glass frit that has been cooled to - 25 °C in a freezer. Air was drawn through the filter for about 5 minutes of drying and powder X-ray diffraction was performed. A powder X-ray diffraction pattern of Form C as shown in Figure 4 was obtained. The yield was about 300 mg.

Example 8: Preparation of Form C (with n-heptane).

966 mg of amorphous anacetrapib was weighed into a 15 ml glass vial and dissolved in 4.5 ml of heptane at 40 °C. This solution was slowly cooled to 10 °C; however, an amorphous precipitate was obtained. After temperature cycling between 10 °C and 20 °C the vial was placed in a refrigerator at about 4 °C for three days. Visual inspection suggested that the sample was partially crystalline, then the suspension was stirred at 2 °C for about 20 hours until it became well stirable and was completely crystalline. After further cooling to -34 °C and stirring at this temperature for about three hours before the crystalline material was separated by filtration with pre-cooled glass frit. Air was drawn through the filter for about 5 minutes for drying and powder X-ray diffraction was performed. A powder X-ray diffraction pattern of Form C as shown in Figure 5 was obtained. Investigation of the sample by thermogravimetry coupled with FTIR spectroscopy revealed a mass loss of less than 0.8% upon heating to 200 °C at a heating rate of 10K/min. The yield was about 550 mg.

Example 9: Preparation of Form C (with n-pentane).

1.08 g amorphous anacetrapib was weighed into a 15 ml glass vial and dissolved in 4.0 ml pentane at room temperature. This solution was slowly cooled to 4 °C and stirred at this temperature. After about three hours a suspension was obtained and stirring was continued at 0 °C overnight. On the next day the temperature was reduced to -5 °C and stirring was continued for about one hour before the solid product was separated by filtration with a glass frit that has been cooled to -25 °C in a freezer. Air was drawn through the filter for about 5 minutes for drying. The yield was about 600 mg.

Powder X-ray diffraction was performed and a powder X-ray diffraction pattern of Form C identical to that shown in Figure 4 was obtained. The sample from the X-ray diffraction sample holder was recovered and used for analysis by thermogravimetry coupled with FT-IR and DSC. The thermogravimetric analysis showed that the sample is essentially free of residual solvent; i.e., no weight loss was observed upon heating to 250 °C. The DSC analysis show no signal at the glass transition temperature at 40 °C, but it shows a melting peak near 83 °C with an enthalpy of fusion of 24 J/g as

shown in Figure 6. The signal to noise ratios of the strongest peaks of the powder X-ray diffraction pattern were evaluated and the following results were found:

| Peak location (2θ) | 7.4° | 17.5° | 19.1° | 20.9° |
|---|---|---|---|---|
| Peak counts | 33293 | 5788 | 9329 | 16555 |
| Baseline counts | 700 | 1540 | 2211 | 3100 |
| Noise level counts | ~100 | ~100 | ~100 | ~100 |
| Signal to noise | 652 | 85 | 142 | 269 |

Example 10: Storage of Form C at room temperature.

About 50 mg of Form C according to Example 7 was prepared into circular silicon X-ray diffraction sample holder with 12 mm diameter and 0.5 mm depth and powder X-ray diffraction is performed. After the measurement the sample holder was kept at room temperature and humidity in a cell culture plate covered with a glass plate for twenty days. After twenty days the sample was again investigated by powder X-ray diffraction which confirmed that the crystalline form was stable and no significant change of the powder X-ray diffraction pattern was found as shown in Figure 7. The yield was about 50 mg.

Example 11: Preparation of Form D (with nonafluoro-methoxy-butane).

13.4 mg of amorphous anacetrapib was weighted into a 7 ml glass vial and 150 μl of nonafluoro-methoxy-butane was added. The clear solution was left in the refrigerator at 5 ± 2 °C for two months until crystals were observed by visual inspection. The cap of the vial was opened to let evaporate the remaining amount of solvent. The crystalline material was collected from the bottom of the vial and powder X-ray diffraction was performed. A powder X-ray diffraction pattern as shown in Figure 8 was obtained. Thermogravimetry coupled with Fourier Transform Infrared spectroscopy of the X-rayed sample revealed that the solid contains about 17% of residual nonafluoro-methoxy-butane. The yield was about 10 mg.

Example 12: Preparation of Form D (with methyl-cyclohexane).

515 mg of amorphous anacetrapib was dissolved in 300 μl of methyl cyclohexane and the prepared clear solution was frozen in a bed of dry ice (solid $CO_2$) at -78 °C, and subsequently the glass flask with the frozen solution was connected to a lyophilizer. Lyophilizer type CHRIST, BETA 2-8 LD-2 was operated at a cold trap temperature of -80 °C. After overnight freeze drying the lyophilization was complete, the flask was disconnected and the open flask was placed into a dessicator over liquid methyl cyclohexane. After one day a sample was recovered from the flask and prepared onto a 1.0 mm XRD sample holder, the solid covered with Kapton film, and measured immediately. Powder X-ray diffraction showed that the sample is partially crystalline and a XRD pattern as shown in Figure 9 was obtained. The yield was about 500 mg.

Example 13: Preparation and solid-state (phase) stability of Form D.

156 mg of amorphous anacetrapib was dissolved in 2.0 ml nonafluoro-methoxy-butane in a 4 ml glass vial. The cap was left open to let the solvent evaporate slowly. After one day the solvent was evaporated but a glassy solid residue was obtained. Another 50 μm of nonafluoro-methoxy-butane was added, the cap of the vial was closed and the sample was kept in the refrigerator at 5 ± 2 °C for one day. Light microscopy showed that crystalline material was obtained and powder X-ray diffraction of the sample confirmed the presence of Form D. After powder X-ray diffraction was performed, the sample was recovered from the sample holder and kept in a glass vial for 14 days at room temperature then again tested by powder X-ray diffraction which confirmed that the crystalline form was still identical (as shown in Figure 10). The yield was about 150 mg.

Methods of analysis

The products were analyzed by following methods:

X-Ray powder diffraction method (XRD):

Conditions for obtaining powder X-ray diffraction patterns: the powder X-ray diffraction patterns were obtained by methods known in the art using Bruker D8 Advance powder X-ray diffractometer using $Cu_{K\alpha}$ radiation (tube operating at 40 kV and 40 mA) in the Bragg-Brentano reflection geometry. Generally, the 2θ values are accurate within an error of ± 0.1 - 0.2 °. Data were recorded in the step size was 0.02 °2θ with a step time of 37 seconds. The samples were rotated at 0.5 rps during the measurement. The X-ray diffractometer was equipped with a LynxEye detector. A variable divergence slight was used with a 3° window.

The signal to noise ratio is defined here.

$$S/N = \frac{2H}{h}$$

*H* : Peak height
*h* : Height of baseline noise as visualized in the scheme below.

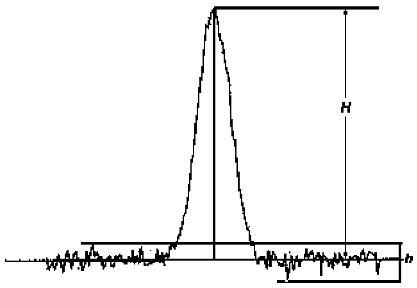


Differential Scanning Calorimetry (DSC): Thermograms were obtained with a Perkin Elmer DSC-7 differential scanning calorimeter. The samples were placed in into gold crucibles that were sealed under nitrogen. The measurements were performed with a heating rate of 10 °C or 20 °C per minute over the temperature range from -50 °C to about 200 °C.
Thermogravimety: Thermogravimetry was performed with a TGA Q5000 from TA Instruments. The samples were heated in open aluminium crucibles from room temperature to variable end temperatures under $N_2$ atmosphere with a heating rate of 10 °C per minute.
Thermogravimetry coupled with Fourier Transform Infrared Spectroscopy (TG-FTIR): TG-FTIR was performed on a Netzsch Thermo-Microbalance TG 209, which is coupled to a Bruker FT-IR Spectrometer Vector 22. The measurements were carried out with aluminum crucibles with a micro pinhole under a nitrogen atmosphere and at a heating rate of 10 °C/min over the range of 25 °C to 250 °C.

## Claims

1. Anacetrapib which is defined by a stable highly crystalline form.

2. The anacetrapib according to claim 1, **characterized by** one of the following X-ray powder diffractogram (XRD) characteristics (i) to (iii):

   (i) at least significant peaks in the XRD pattern at 2θ values of 5.1, 7.7, 19.4, 20.7 and 21.2, respectively exactly or ± 0.2 degrees 2θ at the indicated 2θ values;
   (ii) peaks in the XRD at 2θ values of 5.1, 7.7, 14.3, 17.0, 17.9, 19.4, 20.7, 21.2 and 22.1 in an X-ray powder diffractogram, respectively exactly or ± 0.2 degrees 2θ at the indicated 2θ values;
   (iii) the XRD as shown in Figure 1.

3. The anacetrapib according claim 1 or 2, further **characterized by** differential scanning calorimetry thermogram having endotherm peak in the range of 75°C to 95 °C and having an enthalpy of fusion greater than 22 J/g.

4. A process for the preparation of the anacetrapib according to any one of claims 1 to 3, comprising the steps of:

a) dissolving of amorphous form of anacetrapib in a solvent;
b) adding an anti-solvent to the mixture obtained in step a);
c) optionally stirring the mixture obtained in step b);
d) allowing the mixture obtained in step b) to crystallize.

**5.** The process according to claim 4, wherein step a) is carried out in a solvent, selected from

- organic solvent, preferably alcohol, more preferably ethanol and
- mixture of organic solvent and water, preferably a mixture of alcohol and water, most preferably a mixture of ethanol and water.

**6.** The process according to claim 4 or 5, wherein the solvent is a mixture of organic solvent and water at a ratio of organic solvent, preferably alcohol and more preferably ethanol, to water of about 4 : 1.

**7.** The process according to any one of claims 4 to 6, wherein an anti-solvent in step b) is water.

**8.** The process according to any one of claims 4 to 7, wherein the mixture obtained in step b) is cooled to a temperature lower than room temperature.

**9.** The process according to any one of claims 4 to 8, wherein seed crystals of the crystalline anacetrapib according to claim 2 or 3 are added to the mixture obtained in step b).

**10.** A pharmaceutical composition comprising crystalline anacetrapib according to claim 2 or 3.

**11.** The pharmaceutical composition according to claim 10, for the use in prophylaxis or therapeutic treatment of atherosclerosis, wherein the anacetrapib is in a crystalline non-solvate form.

**12.** The anacetrapib according to claim 1, containing 0 to 30% (w/w) of solvent included in the crystal lattice.

**13.** The anacetrapib according to claim 12, wherein the included solvate is n-pentane.

**14.** The anacetrapib according to claim 12 or 13, **characterized by** one of the following X-ray powder diffractogram (XRD) characteristics (i) to (iii):

(i) at least significant peaks in the XRD pattern at 2θ values of 17.5, 20.2, 20.6, and 20.9, respectively exactly or ± 0.2 degrees 2θ at the indicated 2θ values;
(ii) peaks in the XRD at 2θ values of 7.4, 14.2, 17.5, 20.2, 20.6, 20.9, 22.3 and 22.6 in an X-ray powder diffractogram, respectively exactly or ± 0.2 degrees 2θ at the indicated 2θ values;
(iii) the XRD as shown in Figure 4.

**15.** The anacetrapib according to claim 12, wherein the included solvent is nonafluoro-methoxy-butane.

**16.** The anacetrapib according to claim 12 or 15, **characterized by** one of the following X-ray powder diffractogram (XRD) characteristics (i) to (iii):

(i) at least significant peaks in the XRD pattern at 2θ values of 6.0, 14.0, 16.6, 18.1 and 19.5, respectively exactly or ± 0.2 degrees 2θ at the indicated 2θ values;
(ii) peaks in the XRD at 2θ values of 6.0, 13.7, 14.0, 16.6, 18.1, 19.5, 20.6 and 27.5 in an X-ray powder diffractogram, respectively exactly or ± 0.2 degrees 2θ at the indicated 2θ values;
(iii) the XRD as shown in Figure 8.

**17.** Use of the anacetrapib according to any one of claims 12 to 16 for producing a pharmaceutically active anacetrapib to be used in a pharmaceutically composition.

Figure 1: Powder X-ray diffraction pattern of Form B.

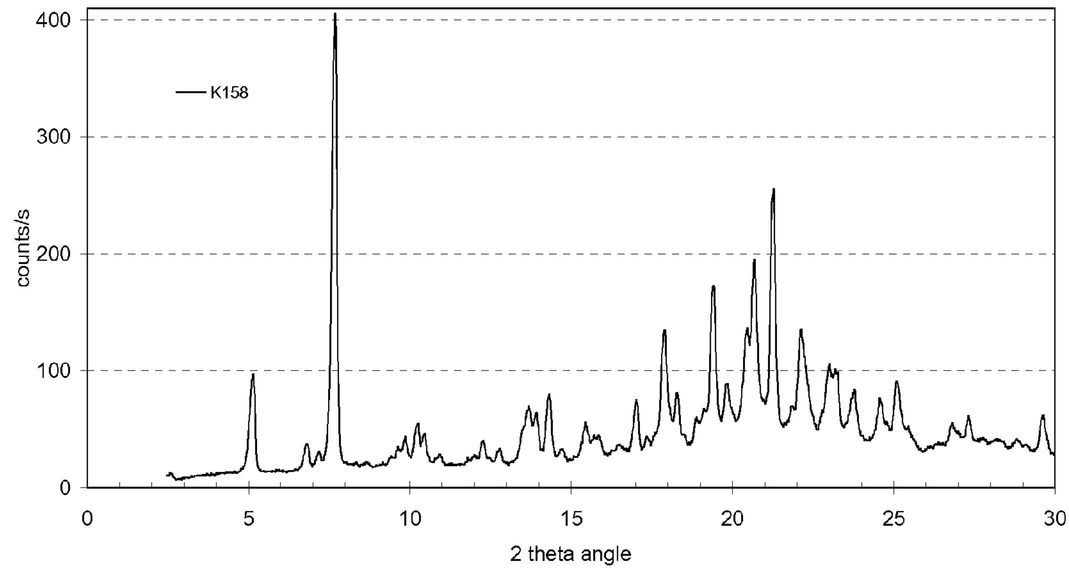


Figure 2: Differential scanning calorimetry thermogram of Form B.

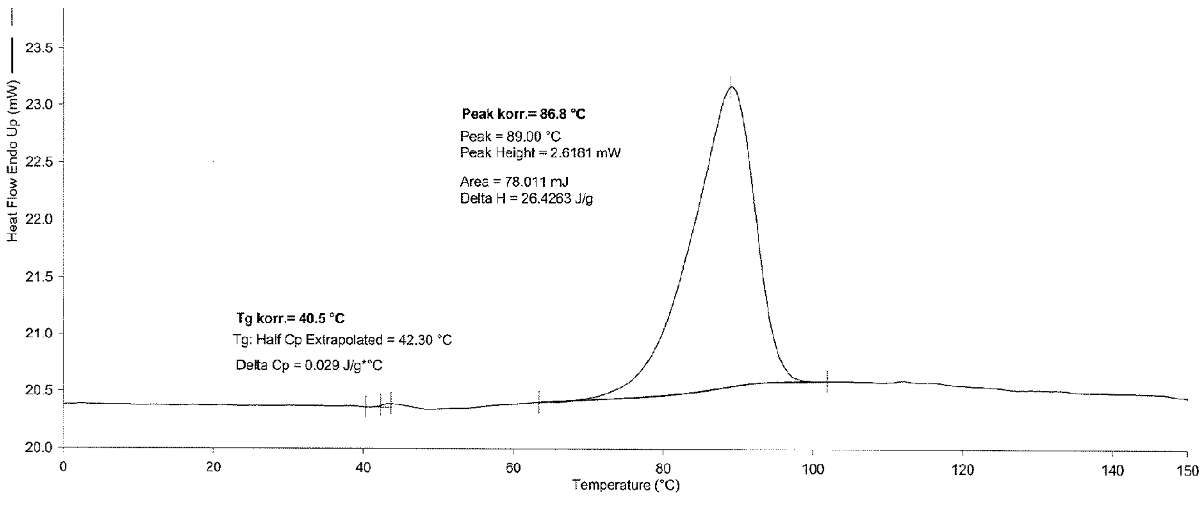

Figure 3: Powder X-ray diffraction pattern of Form B after preparation (lower line) and after storage for 32 days at room temperature (upper line) according to Example 5.

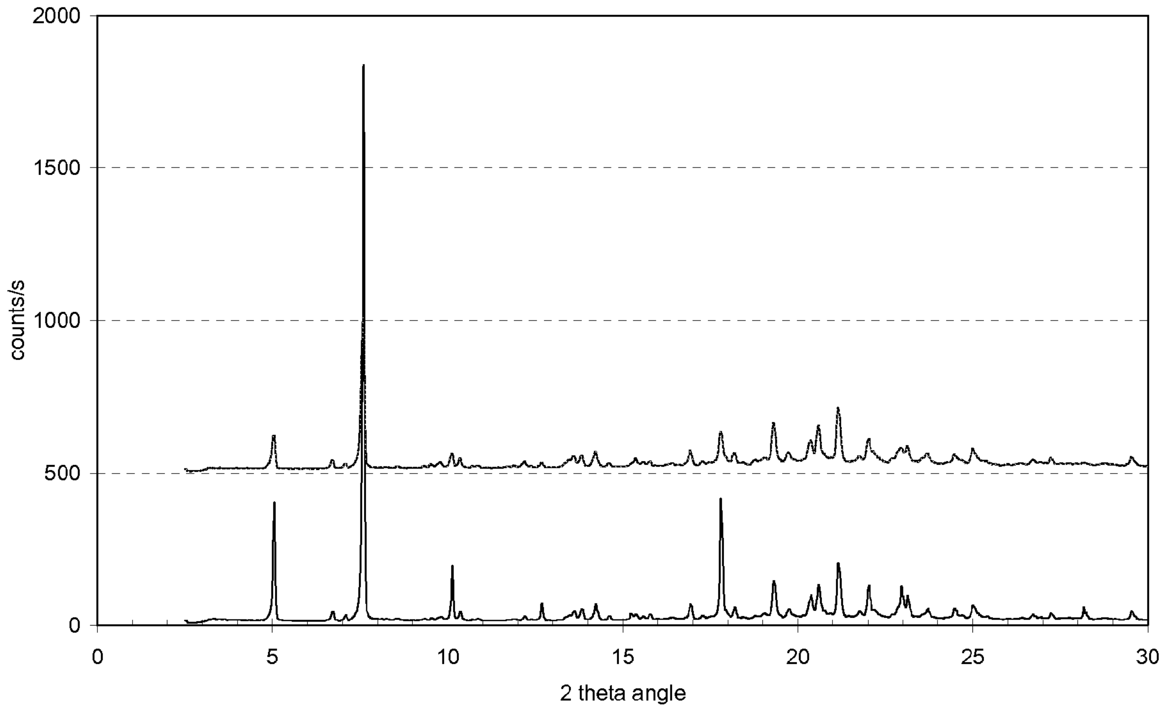


Figure 4: Powder X-ray diffraction pattern of Form C according to Example 7.

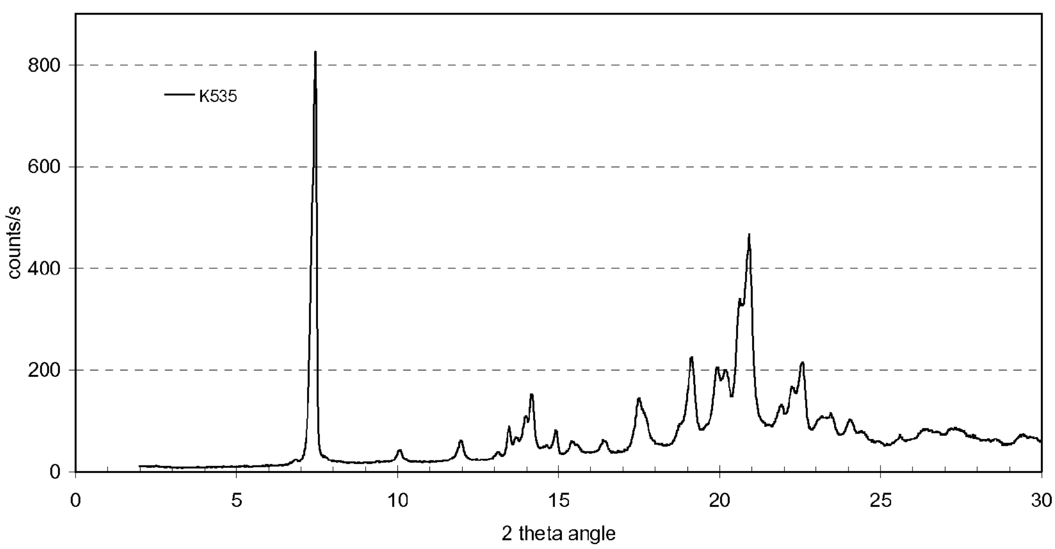

Figure 5: Powder X-ray diffraction pattern of Form C according to Example 8.

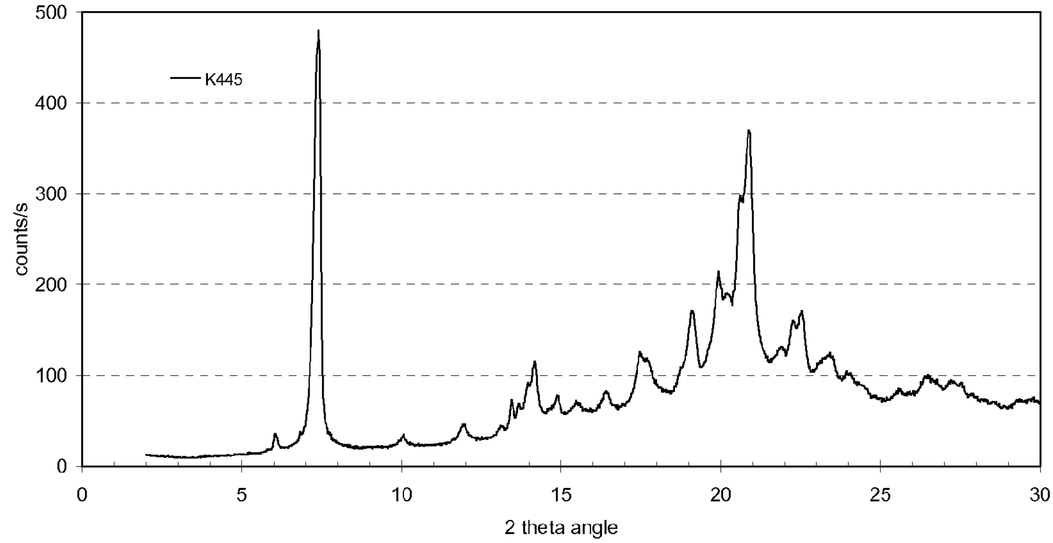


Figure 6: Differential scanning calorimetry thermogram of Form C according to Example 9.

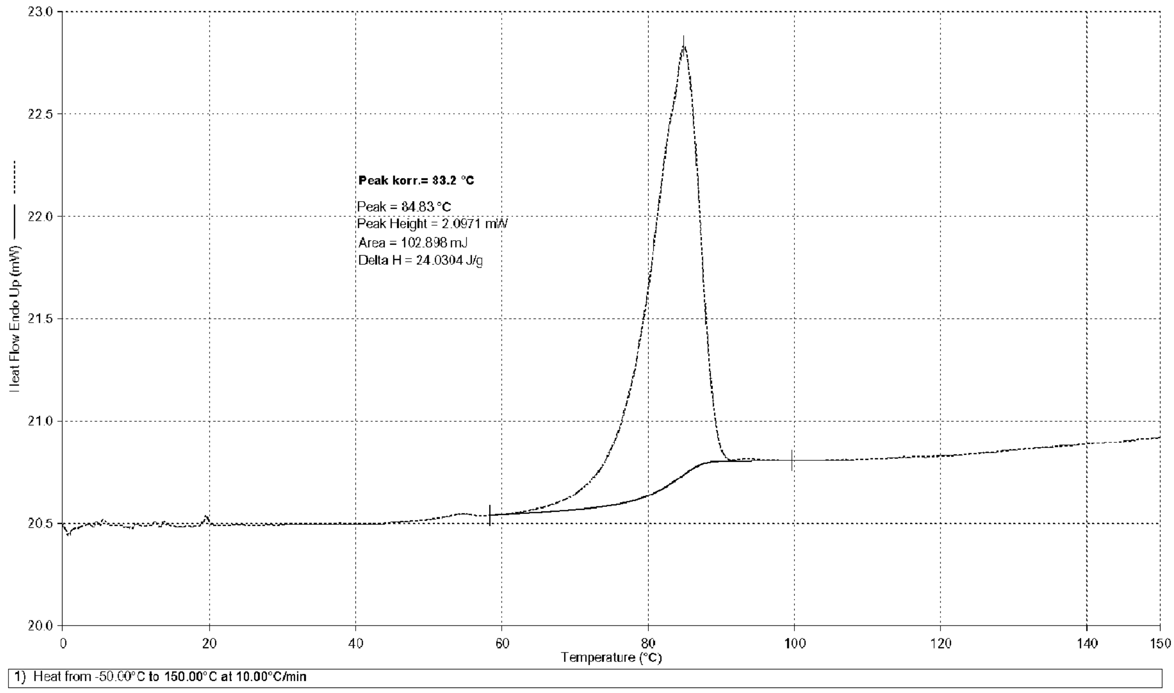

Figure 7: Powder X-ray diffraction pattern of Form C after preparation (bottom line) and after storage for 20 days at room temperature (upper line) according to Example 10.

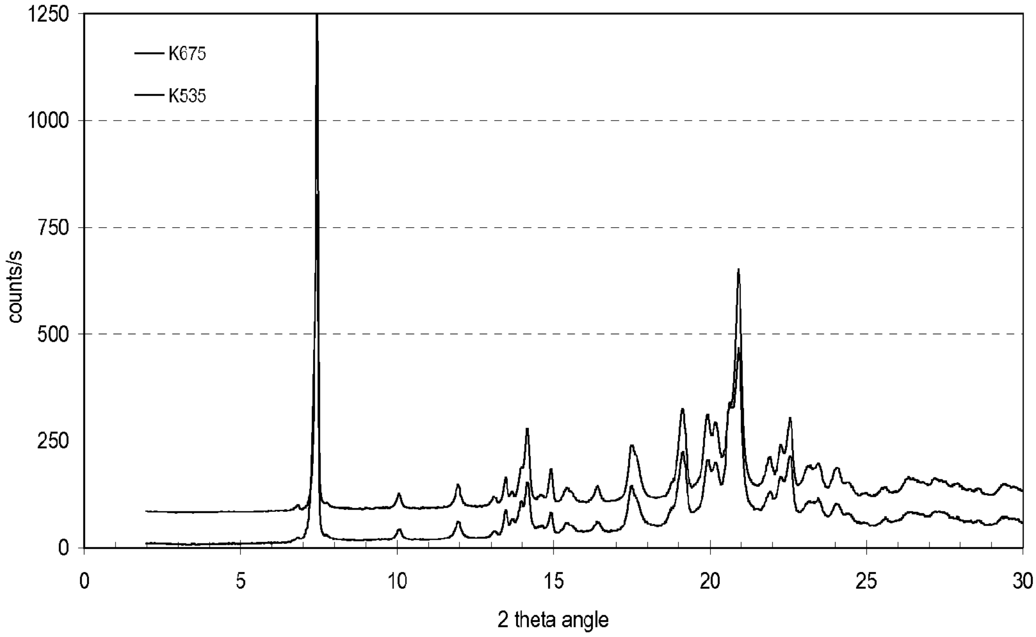


Figure 8: Powder X-ray diffraction pattern of Form D according to Example 11.

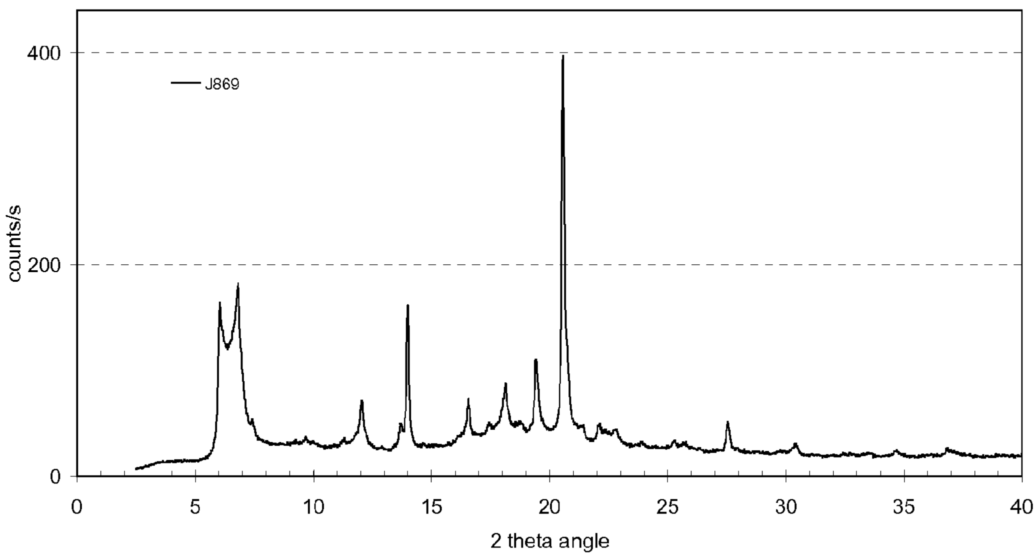

Figure 9: Powder X-ray diffraction pattern of Form D according to Example 12.

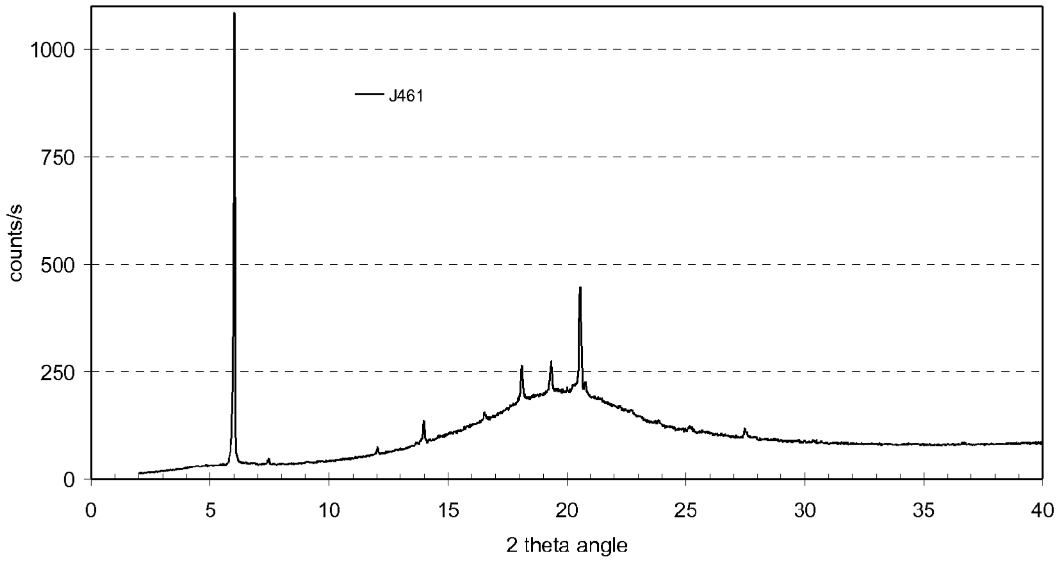


Figure 10: Powder X-ray diffraction pattern of Form D after preparation (lower line) and after storage for two weeks at room temperature (upper line) according to Example 13.

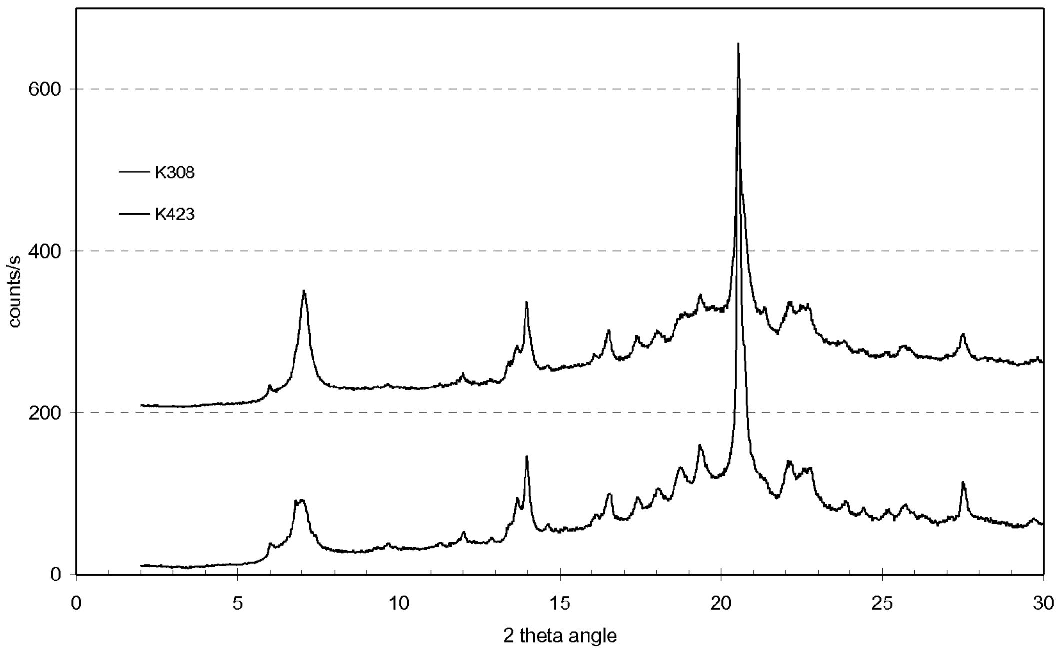

Europäisches Patentamt
European Patent Office
Office européen des brevets

# EUROPEAN SEARCH REPORT

Application Number
EP 10 16 5878

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2007/005572 A1 (MERCK & CO INC [US]; MILLER ROSS A [US]; COTE AARON S [US]) 11 January 2007 (2007-01-11) Abstract; claims 9-30; page 12, line 16 to page 17, line13; figures. ----- | 1-12 | INV. C07D263/20 A61K31/421 A61P9/10 |
| L | BRITTAIN: "X-RAY DIFFRACTION III: PHARMACEUTICAL APPLICATIONS" SPECTROSCOPY, vol. 16, no. 7, July 2001 (2001-07), pages 14-18, XP002606497 Page 15, sections "phase identity of material", "degree of crystallinity". Cited as common general knowledge. ----- | 1-12 | |
| L | BYRN ET AL.: "Solid-State Chemistry of Drugs" 1 January 1999 (1999-01-01), SSCI, SSCI INC, WEST LAFAYETTE, PAGE(S) 82 - 85 , XP002588305 ISBN: 978-0-9670671-0-0 Chapter 5.2: "differential scanning calorimetry". Cited as common general knowledge. ----- | 1-12 | TECHNICAL FIELDS SEARCHED (IPC) |
| L | CAIRA: "CRYSTALLINE POLYMORPHISM OF ORGANIC COMPOUNDS" TOPICS IN CURRENT CHEMISTRY, vol. 198, 1 January 1998 (1998-01-01), pages 163-208, XP001156954 The whole document. Cited as common general knowledge. ----- | 1-12 | C07D A61K A61P |

~~The present search report has been drawn up for all claims~~

1

| Place of search | Date of completion of the search | Examiner |
|---|---|---|
| Munich | 25 October 2010 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number

EP 10 16 5878

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  2, 3, 10, 11 (completely); 1, 4-9, 12 (partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 10 16 5878

The Search Division considers that the present European patent application does not comply with the requirements of unity of invention and relates to several inventions or groups of inventions, namely:

1. claims: 2, 3, 10, 11(completely); 1, 4-9, 12(partially)

   directed to anacetrapib defined by a stable highly crystalline form and the characteristics of claims 2 and/or 3 (i.e. form B of antracepib according to the description), and subject-matter referring to it;

   ---

2. claims: 13, 14(completely); 1, 4-9, 12, 17(partially)

   directed to anacetrapib defined by a stable highly crystalline form and the characteristics of claims 13 and/or 14 (i.e. form C of antracepib according to the description), and subject-matter referring to it;

   ---

3. claims: 15, 16(completely); 1, 4-9, 12, 17(partially)

   directed to anacetrapib defined by a stable highly crystalline form and the characteristics of claims 15 and/or 16 (i.e. form D of antracepib according to the description), and subject-matter referring to it.

   ---

## ANNEX TO THE EUROPEAN SEARCH REPORT ON EUROPEAN PATENT APPLICATION NO. EP 10 16 5878

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2010

| Patent document cited in search report | Publication date | Patent family member(s) | Publication date |
|---|---|---|---|
| WO 2007005572 A1 | 11-01-2007 | AU 2006265975 A1 | 11-01-2007 |
| | | CA 2612142 A1 | 11-01-2007 |
| | | CN 101212966 A | 02-07-2008 |
| | | EP 1901741 A1 | 26-03-2008 |
| | | JP 2009500341 T | 08-01-2009 |
| | | US 2010041724 A1 | 18-02-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 06014413 A
- WO 07005572 A
- WO 2007005572 A